# EUROPEAN PATENT SPECIFICATION

(11) **EP 3 858 318 B1**
(45) Date of publication and mention of the grant of the patent: **21.02.2024**
(21) Application number: 19867461.6
(22) Date of filing: 17.07.2019
(51) Int. Cl.: A61F 13/534, A61F 13/514, A61F 13/53, A61F 13/535, A61F 13/56

(54) **ABSORBENT ARTICLE**
ABSORBIERENDER ARTIKEL
ARTICLE ABSORBANT

(30) Priority: 27.09.2018 JP 2018182563
(43) Date of publication of application: 04.08.2021
(73) Proprietor: DAIO PAPER CORPORATION, Shikokuchuo-shi Ehime 799-0492 (JP); Tohoku University, Sendai-shi, Miyagi 980-8577 (JP)
(72) Inventor: HASEZAWA, Atsuko, Sakura-shi, Tochigi 329-1411 (JP); HOKKIRIGAWA, Kazuo, Sendai-shi, Miyagi 980-8577 (JP); YAMAGUCHI, Takeshi, Sendai-shi, Miyagi 980-8577 (JP); SHIBATA, Kei, Sendai-shi, Miyagi 980-8577 (JP)
(74) Representative: Epping - Hermann - Fischer
(86) International application number: PCT/JP2019/028149
(87) International publication number: WO 2020/066234

(56) References cited:
- WO-A1-2011/102427
- WO-A1-2014/125858
- JP-A- 2001 355 173
- JP-A- 2010 507 440
- JP-A- 2012 135 445
- JP-A- 2013 138 692
- JP-U- H04 128 729

## Description

### TECHNICAL FIELD

The present invention relates to an absorbent article.

### BACKGROUND ART

Known absorbent articles such as a sanitary napkin, a pantiliner, and an incontinence pad are made by sandwiching an absorbing part between a permeable top sheet and an impermeable back sheet.

Also, there is an absorbent article that is configured to be deformable according to movement of the body of a wearer. For example, Patent Document 1 describes a configuration including a shrinkable absorption structure disposed between a front-side sheet and a back-side sheet. The absorption structure includes a large number of block-shaped absorbers that are independent of each other; and the back-side sheet has stretchability at least in the longitudinal direction, is in a contracted state when not stretched, and stretches at a predetermined ratio in the longitudinal direction in response to a predetermined tensile load (e.g., claim 1).

### [RELATED-ART DOCUMENT]

### [PATENT DOCUMENT]

[Patent Document 1] Japanese Patent No. 5779414 [Patent document 2] WO2014/12585
[Patent document 3] WO2011/102427

### DISCLOSURE OF INVENTION

### PROBLEMS TO BE SOLVED BY THE INVENTION

With the configuration disclosed in Patent Document 1, the entire absorbent article has stretchability and therefore can expand and contract along with the expansion and contraction of underwear caused by the movement of the body. However, the configuration disclosed in Patent Document 1 does not take into account the friction between the absorbent article and the skin, which may occur when the body of a wearer of the absorbent article moves during, for example, walking. Such friction may reduce the wear comfort and, in some cases, may cause the wearer to feel, for example, itchiness or pain. Patent Documents 2 and 3 disclose further absorbent articles with friction reducing layer.

In view of the above problems, one object of the present invention is to provide an absorbent article that is more comfortable to wear and configured to prevent friction between the absorbent article and the skin which may occur while the absorbent article is worn.

### MEANS FOR SOLVING THE PROBLEMS

According to a first aspect of the present invention, an absorbent article includes a permeable top sheet, an impermeable back sheet, an absorbing part disposed between the top sheet and the back sheet, and a friction reducing layer disposed between the back sheet and the absorbing part. The friction reducing layer is at least one selected from a group consisting of: a sheet-like part inserted between the back sheet and the absorbing part, a friction reducing treatment being applied to at least one surface of the sheet-like part; a sheet-like part that is inserted between the back sheet and the absorbing part, and is formed of at least one material
selected from a group consisting of silicone resin, fluororesin, or polyacetal resin; and a layer formed by applying the friction reducing treatment to at least one of the back sheet and the absorbing part. The friction reducing treatment is a process of applying at least one of silicone resin, fluororesin, polycetal resin, talc, silica, and mica. The friction between the friction reducing layer and the back sheet or the friction between the friction reducing layer and the absorbing part is less than the friction between the absorbing part and the back sheet.

### ADVANTAGEOUS EFFECT OF THE INVENTION

An aspect of the present invention makes it possible to provide an absorbent article that is more comfortable to wear and configured to prevent friction between the absorbent article and the skin which may occur while the absorbent article is worn.

### BRIEF DESCRIPTION OF THE DRAWINGS

FIG. 1 is a partially-broken plan view of an absorbent article according to an embodiment of the present invention;
FIG. 2 is a cross-sectional view taken along line I-I of the absorbed article of the embodiment of the present invention;
FIG. 3 is a drawing illustrating a variation of a friction reducing layer of the absorbent article according to the embodiment of the present invention;
FIG. 4 is a drawing illustrating a variation of a friction reducing layer of the absorbent article according to the embodiment of the present invention;
FIG. 5 is a drawing illustrating a variation of the absorbent article according to the embodiment of the present invention; and
FIG. 6 is a drawing illustrating a variation of the absorbent article according to the embodiment of the present invention.

### DESCRIPTION OF EMBODIMENTS

An embodiment of the present invention is described below with reference to the accompanying drawings. Unless otherwise mentioned, the same reference number is assigned to the same component or corresponding components throughout the drawings, and repeated descriptions of those components may be omitted. Also, the drawings are schematic diagrams for facilitating the understanding of the invention, and some dimensions may be greater than or less than the actual dimensions.

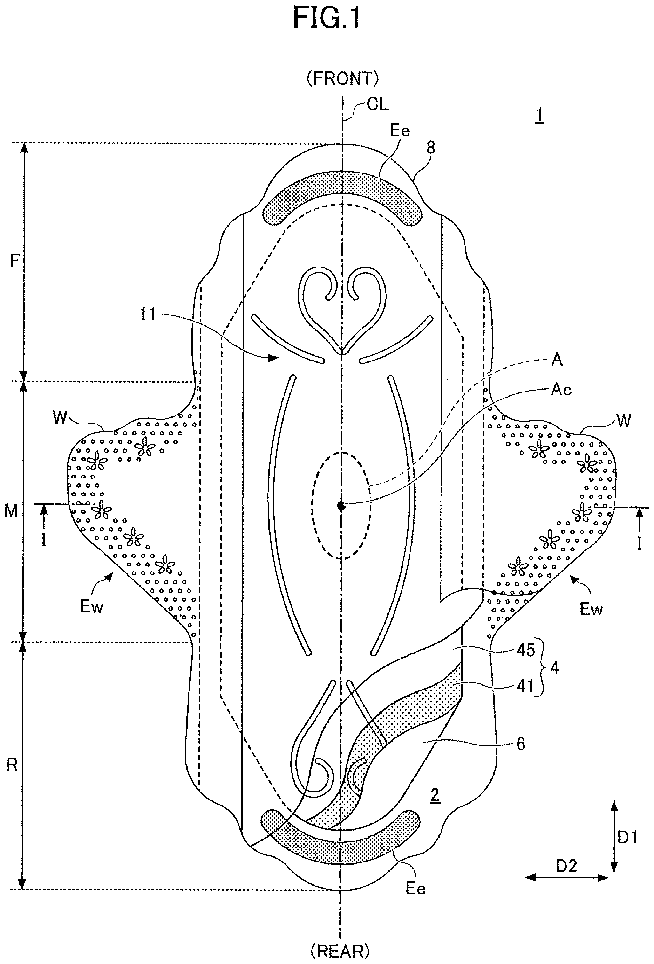

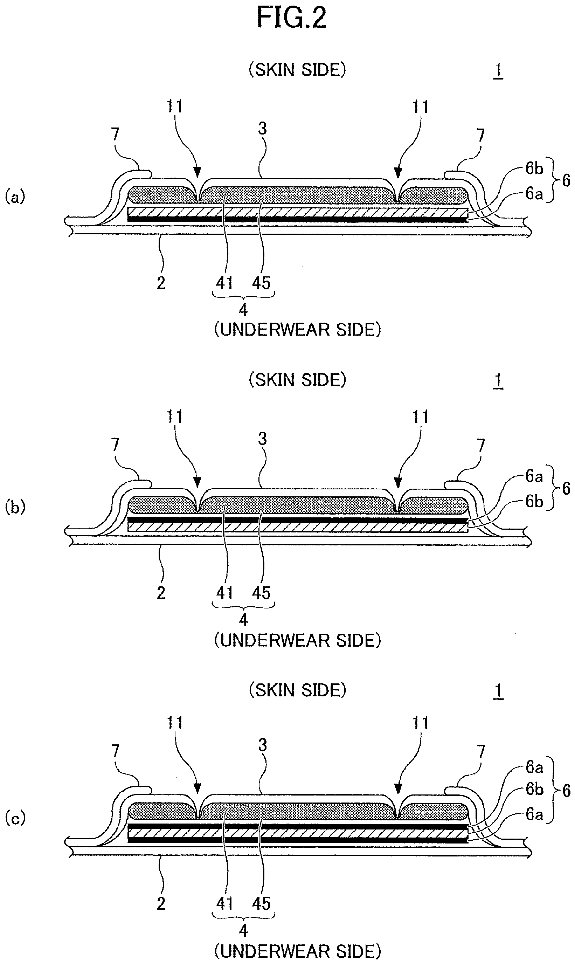

FIG. 1 is a partially-broken plan view of an absorbent article 1 according to an embodiment of the present invention. FIG. 2 (a) is a cross-sectional view taken along line I-I of FIG. 1. As illustrated in FIG. 1 and FIG. 2 (a), the absorbent article 1 includes a body (absorbent article body) 8 including an impermeable back sheet 2, a permeable top sheet 3, and an absorbing part 4 provided between the sheets 2 and 3. The absorbent article 1 is worn such that the back sheet 2 (underwear side) of the body 8 is fixed to underwear such as shorts and the top sheet 3 (skin side) contacts the skin. An antislip part (not illustrated in FIG. 1) for fixing the absorbent article 1 to the underwear may be provided on the underwear side of the back sheet 2.

The absorbent article body 8 is a portion of the absorbent article 1 excluding wings W described later. As a whole, the body 8 has an elongated shape that has a predetermined length in the longitudinal direction (the front-back direction, i.e., a first direction D1 in the figure) and a predetermined width in the width direction (a second direction D2 in the figure) orthogonal to the longitudinal direction D1. Although the width of the body 8 is substantially constant in the illustrated example, the width of the body 8 may vary along the longitudinal direction D1. The absorbent article 1 may have a shape and a structure that are substantially axisymmetric with respect to a center line (longitudinal center line) CL extending in the longitudinal direction.

In the example of FIG. 1, both of the back sheet 2 and the top sheet 3 extend in the longitudinal direction D1 from the front end to the rear end of the absorbent article 1, and the width of the top sheet 3 is less than the width of the back sheet 2. Side nonwoven fabrics 7 extending in the longitudinal direction D1 along the lateral ends of the absorbing part 4 overlap the skin side of the top sheet 3.

The body 8 of the absorbent article 1 includes a region (body fluid outlet region) A that faces a body fluid outlet such as the vaginal opening of a wearer wearing the absorbent article 1, and the body fluid outlet region A has a center Ac. The center Ac is located on the longitudinal center line CL and near the center of the body fluid outlet region A in the longitudinal direction D1. The absorbent article 1 includes a middle region M including the body fluid outlet region A, a front region F next to the front end of the middle region M, and a rear region R next to the rear end of the middle region M.

The middle region M may include wings W that extend from the lateral sides of the middle region M and are used to reliably fix the absorbent article 1 to the underwear when the absorbent article 1 is worn. The front region F extends from positions corresponding to or near the front starting points of the wings W to the front end, and the rear region R extends from positions corresponding to or near the rear starting points of the wings W to the rear end. Also, when the wings W are provided, in the longitudinal direction D1, the center Ac of the body fluid outlet region A may be located in the midpoint in the length of the lateral edges of the wings W or in the midpoint between the front starting points and the rear starting points of the wings W.

Although the absorbent article 1 in the illustrated example includes the wings W, the absorbent article 1 may be configured to not include the wings W. In this case, the front region F may be a region extending forward from positions corresponding to or near the front starting points of wings W that are assumed to be formed, and the rear region R may be a region extending backward from positions corresponding to or near the rear starting points of wings W that are assumed to be formed.

The overall length of the absorbent article 1 is preferably between 150 and 450 mm, and more preferably between 170 and 290 mm.

The absorbing part 4 is provided between the top sheet 2 and the back sheet 3 so as not to be exposed to the outside. For example, as illustrated in FIG. 1, the absorbing part 4 may be configured and disposed to fit in the back sheet 2 in plan view, i.e., not to extend beyond the back sheet 2. Also, the absorbing part 4 may be configured and disposed to fit in a region where the top sheet 3 and the side nonwoven fabrics 7 are provided.

At the front and rear ends of the absorbing part 4, end seals Ee may be formed by bonding the outer edges of the back sheet 2 and the top sheet 3 with an adhesive such as a hot melt or by a bonding technique such as heat sealing or ultrasonic sealing. Also, at the lateral ends of the absorbing part 4, the side nonwoven fabrics 7 and the back sheet 2 may be bonded to each other by a bonding technique as described above to form the wings W. Embossments Ew may be formed on the wings W to increase the bonding strength and improve the design.

Thus, the entire periphery of the absorbing part 4 may be sealed. This makes it possible to prevent a body fluid absorbed by the absorbing part 4 from leaking from the side of the absorbing part 4 and also make it possible to improve the shape stability of the absorbent article 1. The strength of the end seals Ee is preferably greater than that of the side seals. This allows the back sheet 2 and the absorbing part 4 to slide relative to each other as described later, and also makes it possible to stabilize the shape of the absorbent article 1.

The back sheet 2 may be formed of a sheet material having at least a waterproof property such as an olefinic resin sheet made of polyethylene or polypropylene. Also, the back sheet 2 may be implemented by a laminated nonwoven fabric formed by stacking a nonwoven fabric on, for example, a polyethylene sheet, or a laminated sheet that is made substantially impermeable by stacking a waterproof film between nonwoven fabrics. Further, a sheet having moisture permeability is more preferably used to prevent stuffiness. As the water-impermeable and moisture-permeable sheet, a microporous sheet is preferably used. A microporous sheet is obtained by melt-mixing an inorganic filler with an olefin resin such as polyethylene or polypropylene to form a sheet, and by stretching the sheet uniaxially or biaxially.

The top sheet 3 is a permeable sheet through which body fluids such as blood, discharge, and urine can permeate rapidly. The top sheet 3 is preferably formed of a porous or nonporous nonwoven fabric or a porous plastic sheet. The nonwoven fabric may be formed by using one of or a combination of two or more of the following materials: olefins such as polyethylene and polypropylene, synthetic fibers such as polyester and polyamide, regenerated fibers such as rayon and cupra, a blend of these fibers, and natural fibers such as cotton. The nonwoven fabric may be produced by, for example, spunlacing, spunbonding, thermal bonding, melt-blowing, or needle punching. Among these production methods, spunlacing is preferable to produce a flexible nonwoven fabric, spunbonding is preferable to produce a nonwoven fabric with good draping characteristics, and thermal bonding is preferable to produce a bulky and soft nonwoven fabric. Also, bicomponent fibers may be used for the synthetic fibers. Examples of bicomponent fibers include a core-in-sheath fiber including a high-melting-point fiber as a core and a low-melting-point fiber as a sheath, a side-by-side fiber, and a split fiber.

As illustrated in FIG. 1, the absorbing part 4 may include an absorber 41 that essentially performs the function of absorbing a body fluid and an enveloping sheet 45 that envelopes the absorber 41. The material of the enveloping sheet 45 may be, for example, but is not limited to, crepe paper or a nonwoven fabric. Enveloping the absorber 41 with the enveloping sheet 45 makes it possible to prevent the absorber 41 from wrinkling or cracking. The enveloping sheet 45 may be implemented by either uncolored (i.e., white) crepe paper or nonwoven fabric or colored crepe paper or nonwoven fabric.

The absorber 41 may be implemented by any material that can absorb and hold a body fluid, and preferably includes cotton pulp and an absorbent polymer. Examples of absorbent polymers include superabsorbent polymer (SAP) granular powder (SAP), superabsorbent fibers (SAF), and a combination of them. Examples of the pulp include chemical pulp obtained from wood, cellulose fibers such as dissolving pulp, and artificial cellulose fibers such as rayon and acetate. Hardwood and softwood may be used as the materials of the chemical pulp, but softwood is preferably used because of its long fiber length. The absorber 41 may be produced by fiber stacking or an air-laid method.

Synthetic fibers may also be added to the absorber 41. Examples of materials of the synthetic fibers include polyolefin such as polyethylene and polypropylene, polyester such as polyethylene terephthalate or polybutylene terephthalate, polyamide such as nylon, and a copolymer of these materials. Also, the synthetic fibers may be formed by mixing two or more of these materials. Also, bicomponent fibers may be used for the synthetic fibers. Examples of bicomponent fibers include a core-in-sheath fiber including a high-melting-point fiber as a core and a low-melting-point fiber as a sheath, a side-by-side fiber, and a split fiber. Further, a material obtained by surface-treating hydrophobic fibers with a hydrophilic agent to have an affinity to body fluids may be used as the synthetic fibers.

The absorbing part 4 may be implemented by a polymer sheet including a superabsorbent polymer sandwiched in a predetermined region between a skin-side sheet and an underwear-side sheet.

The thickness of the absorbing part 4 may be between 0.3 and 30 mm, and is preferably between 1.0 and 15 mm. The absorber 41 does not necessarily have a uniform thickness in its entirety, and the body fluid outlet region A, a region near the body fluid outlet region A, and a central portion of the rear region in the width direction may be bulging.

The side nonwoven fabrics 7 may be implemented by a water-repellent nonwoven fabric or a hydrophilically-treated nonwoven fabric. For example, a water-repellent nonwoven fabric coated with a silicon- or paraffin-repellent is preferably used to more prevent penetration of body fluids such as blood and discharge or to improve the feel. Also, to improve the absorbency of the wings W for absorbing, for example, menstrual blood, the wings W are preferably formed of a hydrophilically-treated nonwoven fabric. As a type of the nonwoven fabric, an air-through nonwoven fabric, which is soft and unlikely to be creased and wrinkled, is preferably used.

As illustrated in FIG. 1, compressed grooves 11 (which are also referred to as fit embossments) dented from the top sheet 3 toward the back sheet 2 may be formed in the body 8. The compressed grooves 11 may be formed by passing a laminated structure obtained by stacking the top sheet 3 on the absorbing part 4 through a gap between a pair of pressing rollers. For example, the laminated structure may be pressed by using a patterned roller disposed to face the top sheet 3 and a flat roller disposed to face the absorbing part 4. With the compressed grooves 11 formed, the top sheet 3 and the absorbing part 4 are joined by the compressed grooves and can deform and move together while the absorbent article 1 is worn.

A friction reducing layer 6 is disposed between two contacting objects and has a function to reduce friction or a frictional force between the two objects. In the present embodiment, the friction reducing layer 6 is configured such that at least one of the friction between the friction reducing layer 6 and the absorbing part 4 and the friction between the friction reducing layer 6 and the back sheet 2 becomes less than the friction between the absorbing part 4 and the back sheet 2.

As illustrated in FIG. 1 and FIG. 2 (a), the friction reducing layer 6 is provided between the back sheet 2 and the absorbing part 4. This configuration makes it possible to reduce the friction between the back sheet 2 and the absorbing part 4. Here, when the absorbing part 4 has a configuration where the absorber 41 is enveloped by the enveloping sheet 45 as described above, the friction reducing layer 6 may be configured such that at least one of the friction between the friction reducing layer 6 and the enveloping sheet 45 and the friction between the friction reducing layer 6 and the back sheet 2 becomes less than the friction between the enveloping sheet 45 and the back sheet 2. Also, when a polymer sheet is used as the absorbing part 4, the friction reducing layer 6 may be configured such that at least one of the friction between the friction reducing layer 6 and an underwear-side sheet of the polymer sheet and the friction between the friction reducing layer 6 and the back sheet 2 becomes less than the friction between the underwear-side sheet of the polymer sheet and the back sheet 2. Thus, the friction reducing layer 6 can reduce the friction, more specifically the sliding friction, between a surface of a sheet or a layer, which would have been in contact with the back sheet 2 unless the friction reducing layer 6 is provided, and the skin-side surface of the back sheet 2.

While the absorbent article 1 is worn, there may be a case where the wearer moves the legs largely or continuously in, for example, a walking motion. In such a case, the underwear may be deformed by the force and movement of the legs, and the back sheet 2 may also deform and move along with the deformation of the underwear. Here, if the back sheet 2 and the absorbing part 4 are stuck together by friction or with an adhesive, the structure including the absorbing part 4 and components above the absorbing part 4 (e.g., the absorbing part 4, the top sheet 2, and any additional component) may deform and move along with the back sheet 2, and therefore moves relative to the skin of the wearer facing the absorbent article. As a result, the skin may be rubbed by the surface of the top sheet 2 of the absorbent article 1. This rubbing may reduce the wear comfort and cause the wearer to feel itchiness or pain.

In contrast, in the present embodiment, the friction reducing layer 6 is provided between the back sheet 2 and the absorbing part 4 so that the back sheet 2 and the absorbing part 4 can slide relative to each other in the in-plane direction and the back sheet 2 and the absorbing part 4 are less likely to move along with each other. This configuration makes it possible to prevent or reduce the movement of the absorbing part 4 and the top sheet 3 disposed on the absorbing part 4 relative to the skin of the wearer even when the back sheet 2 deforms and moves along with the deformation of the underwear. Accordingly, this configuration makes it possible to reduce the friction between the top sheet 2 of the absorbent article 1 and the skin and thereby improve the wear comfort.

The friction reducing layer 6 may be formed of any material that can reduce the friction between the back sheet 2 and the absorbing part 4 and enable them to slide relative to each other in a direction along the contact surface. For example, the friction reducing layer 6 may be a sheet-like part including a base sheet 6b and a friction reduced portion 6a that is formed by applying a friction reducing treatment to at least a part of one surface of the base sheet 6b.

As illustrated in FIG. 2 (a), in the friction reducing layer 6 formed as a sheet-like part, the friction reduced portion 6a may be formed on the underwear side of the base sheet 6b. This configuration enables the skin-side surface of the back sheet 2 and the underwear-side surface of the friction reducing layer 6 to smoothly slide relative to each other.

In the example of FIG. 2 (a), the skin-side surface (which faces the absorbing part 4) of the friction reducing layer 6 may be or may not be bonded to the absorbing part 4. Bonding the friction reducing layer 6 to the absorbing part 4 makes it possible to prevent the friction reducing layer 6, which is a separate component, from being misaligned even when a large force is applied to the absorbent article 1.

FIG. 2 (b) and FIG. 2 (c) illustrate variations of the friction reducing layer 6 that is a sheet-like part. As illustrated in FIG. 2 (b), in the friction reducing layer 6, the friction reduced portion 6a may be formed on the skin side of the base sheet 6b. With the example of FIG. 2 (b), the underwear-side surface of the absorbing part 4 and the skin-side surface of the friction reducing layer 6 can be smoothly slid relative to each other.

Also in the example of FIG. 2 (b), the underwear side (which faces the back sheet 2) of the friction reducing layer 6 may be either bonded or not bonded to the back sheet 2. Bonding the friction reducing layer 6 to the back sheet 2 makes it possible to prevent the friction reducing layer 6, which is a separate component, from being misaligned even when a large force is applied to the absorbent article 1.

Also, as illustrated in FIG. 2 (c), the friction reduced portion 6a may be provided on each of the underwear side and the skin side of the base sheet 6b. This makes it possible to reduce friction on both sides of the base sheet 6b. In this example, the friction reducing layer 6 slides relative to one of the skin-side surface of the back sheet 2 and the underwear-side surface of the absorbing part 4 which has lower sliding friction with the friction reduced portion 6a. This example enables the friction reducing layer 6 to slide relative to at least one of the back sheet 2 and the absorbing part 4, and thereby makes it possible to select the materials of the back sheet 2 and the absorbing part 4 from a greater number of candidates.

Here, the friction reducing treatment may be performed, for example, by applying a material (composition) including a substance capable of imparting slipperiness or a material composed of a substance capable of imparting slipperiness. Examples of substances capable of imparting slipperiness include organic substances such as silicone resin, fluororesin, and polyacetal resin, and inorganic substances such as talc, silica, and mica. The form of the material to be applied may be, for example, but is not limited to, solution, emulsion, suspension, or powder. When the material is applied in the form of powder, the material is preferably applied to the back sheet 2. When the friction reducing layer 6 formed as a sheet-like part as illustrated in FIG. 2 (a) through FIG. 2 (c) is used, the amount of the friction reduced portion 6a formed by the friction reducing treatment may be about 10.0 to about 30.0 g/m² (in dry state).

Also, the material including a substance capable of imparting slipperiness or the material composed of a substance capable of imparting slipperiness may be in the form of powder. In this case, silicone powder having excellent dispersibility is preferably used.

The base sheet 6b may be implemented by a film formed of a thermoplastic resin or a combination of thermoplastic resins, a planar body including fibers such as a woven fabric or a nonwoven fabric, a planar body including pulp, or a laminate structure of these materials. Examples of thermoplastic resins used as the materials of the base sheet 6b include olefins such as polyethylene and polypropylene, polyester, and polyvinyl alcohol. Polyethylene is preferable because of its inexpensiveness and good workability.

In the examples illustrated in FIG. 2 (a) through FIG. 2 (c), the friction reducing layer 6 is provided separately from the back sheet 2 and the absorbing part 4. However, in the examples illustrated in FIG. 2(a) and (b), the side of the friction reducing layer 6 on which the friction reduced portion 6a is not formed may be fixed with, for example, a hot melt adhesive. That is, in the example illustrated in FIG. 2 (a), the friction reducing layer 6 may be fixed to the absorbing part 4; and in the example illustrated in FIG. 2 (b), the friction reducing layer 6 may be fixed to the back sheet 2. This configuration makes it possible to prevent the friction reducing layer 6 from being displaced from the original position when the absorbent article 1 is used for a long period of time or if a large force is applied due to hard exercise or when taking out the absorbent article 1, and thereby makes it possible to improve the shape stability of the entire absorbent article 1. If the friction reducing layer 6 is displaced by a large amount, the structure of the absorbent article 1 may be damaged. The present embodiment makes it possible to reduce the possibility of such damage.

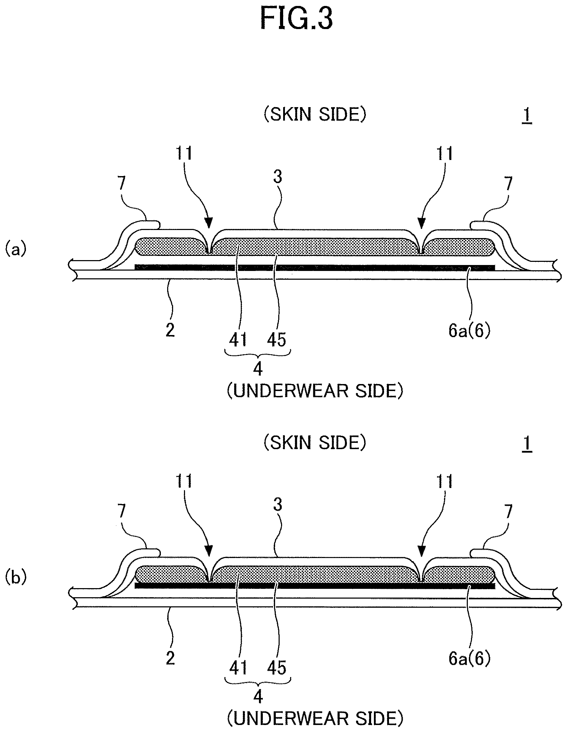

FIG. 3 (a) and FIG. 3 (b) illustrate other variations of the friction reducing layer 6. The friction reducing layer 6 may not necessarily be a sheet-like part that is separate from the back sheet 2 and the absorbing part 4. That is, the friction reducing layer 6 may be formed by applying the friction reducing treatment to at least one of the back sheet 2 and the absorbing part 4 and thereby forming the friction reduced portion 6a.

For example, as illustrated in FIG. 3 (a), the friction reducing layer 6 may be implemented by the friction reduced portion 6a that is formed by directly applying a material including a substance capable of imparting slipperiness to the skin-side surface of the back sheet 2. Also, as illustrated in FIG. 3 (b), the friction reduced portion 6a may be formed by directly applying a material including a substance capable of imparting slipperiness to the underwear-side surface of the absorbing part 4. In this case, the amount of the friction reduced portion 6a formed by the friction reducing treatment may be about 10.0 to about 30.0 g/m² (in dry state).

In the examples illustrated in FIG. 3 (a) and FIG. 3 (b), the friction reduced portion 6a can be formed directly on at least one of the back sheet 2 and the absorbing part 4. This configuration eliminates the need to form a sheet as a separate component. This also makes it possible to reduce the number of components and reduce the manufacturing costs. Also, the configuration where the friction reduced portion 6a is directly applied makes it possible to prevent misalignment of the friction reducing layer that is caused due to, for example, long-term use.

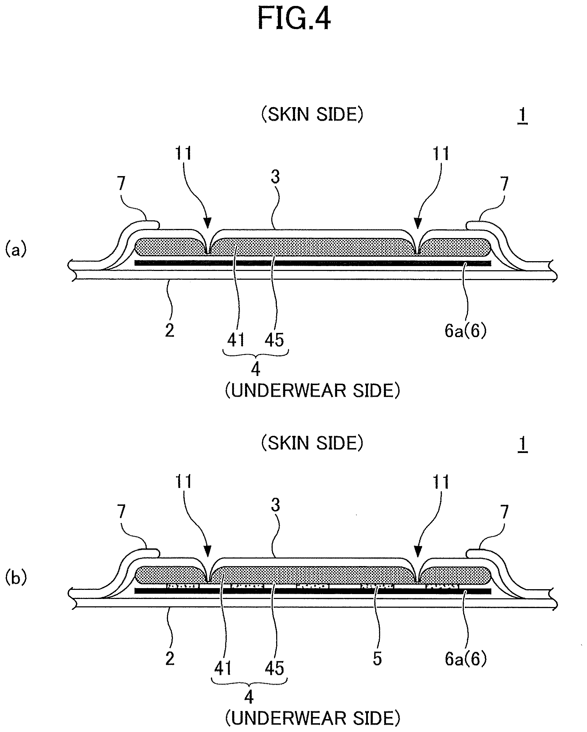

FIG. 4 (a) and FIG. 4 (b) illustrate still other variations of the friction reducing layer 6. As illustrated in FIG. 4 (a), the entire friction reducing layer 6 may be a sheet-like part that is formed of a material including a substance capable of imparting slipperiness such as silicone resin, fluororesin, or polyacetal resin, or of a material composed of a substance capable of imparting slipperiness. In this case, the sheet-like part is preferably formed of silicone resin or fluororesin. In this example, the friction reducing layer 6 can be formed with a substantially uniform material in the thickness direction. With this configuration, even if a surface of the friction reducing layer 6 is peeled off for a certain reason when the absorbent article 1 is worn, the friction reducing effect of the friction reducing layer 6 is less likely to be lost.

Also, depending on the degree of the friction reducing effect of the friction reducing layer 6 and the application of the absorbent article 1, the length of the friction reducing layer 6 may be made less than the length of the absorbing part 4 and/or the width of the friction reducing layer 6 may be made less than the width of the absorbing part 4.

FIG. 4 (b) is a variation of the example of FIG. 4 (a). In the example of FIG. 4 (b), the friction reducing layer 6, which is a sheet-like part formed of a material including a substance capable of imparting slipperiness or a material composed of a substance capable of imparting slipperiness, is bonded via an adhesive 5 such as a hot melt adhesive to the absorbing part 4. This configuration makes it possible to prevent misalignment of the friction reducing layer 6 that is a component separate from the absorbing part 4 and the back sheet 2, and makes it possible to maintain the shape stability of the entire absorbent article 1 even when the absorbent article 1 is used for a long period of time. When the friction reducing layer 6 is bonded, the bonded portion may be either continuous or discontinuous as illustrated in FIG. 3 (d).

In any one of the examples of FIGs. 2 through 4, the friction reduced portion 6a may be formed either continuously or discontinuously, i.e., in multiple regions arranged at intervals. Also, in the examples illustrated in FIGs. 2 through 4, the base sheet 6b itself may be formed either continuously or discontinuously.

Regardless of whether the friction reducing layer 6 is implemented as a sheet-like part (FIGs. 2 and 4) or a friction reduced portion formed directly on the back sheet 2 and/or the absorbing part 4 (FIG. 3), the plan-view area of the friction reducing layer 6 (when two or more separate friction reducing layers are arranged, the sum of their plan-view areas) may be greater than or equal to 40%, preferably 50%, more preferably 60%, and still more preferably greater than or equal to 80% of the plan-view area (100%) of the absorbing part 4. When the plan-view area is greater than or equal to 100%, i.e., when the friction reduced portion 6a covers the entire region where the absorbing part 4 is present or covers a region including the region of the absorbing part 4 and extending beyond the region of the absorbing part 4, the friction reducing effect can be increased, and the back sheet 2 and the absorbing part 4 can more smoothly slide relative to each other in a direction along the contact surface between the back sheet 2 and the absorbing part 4.

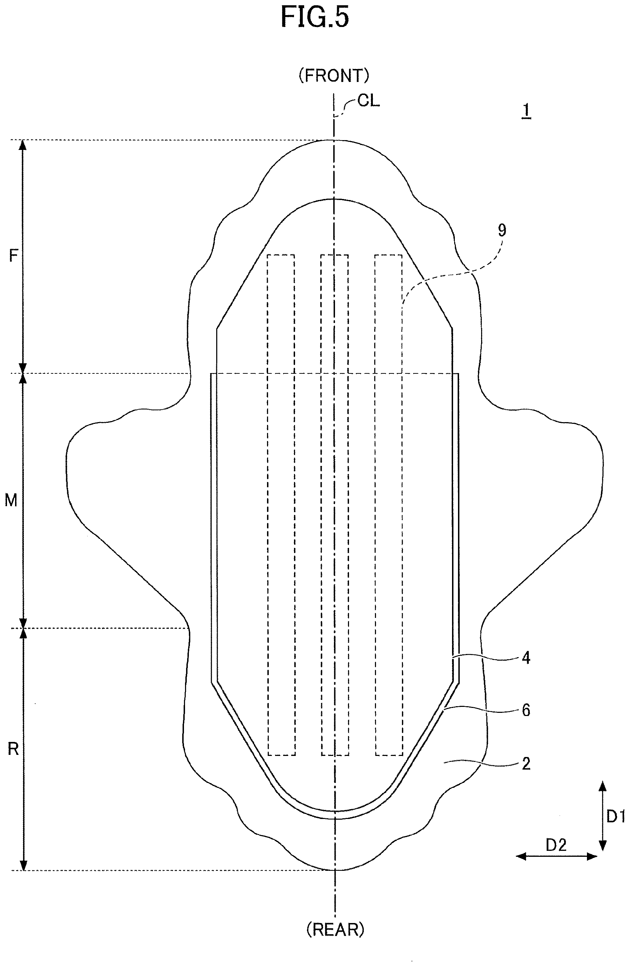

Also, the friction reducing layer 6 may have any size and any shape as long as the friction reducing layer 6 can reduce the friction between the back sheet 2 and the absorbing part 4 and enable them to slide relative to each other in a direction along the contact surface. In the example illustrated in FIG. 1 and FIG. 2 (a), the friction reducing layer 6 has the same size and shape as those of the absorbing part 4, and the friction reducing layer 6 and the absorbing part 4 are arranged such that their contours are substantially aligned with each other. However, the size of the friction reducing layer 6 in plan view may be greater than the absorbing part 4 unless the friction reducing layer 6 prevents bonding the back sheet 2, the top sheet 3, and the side nonwoven fabrics 7 to each other at the periphery of the absorbing part 4. That is, the friction reducing layer 6 may protrude from the absorbing part 4 (FIG. 5). In other words, in the longitudinal direction D1 and/or the width direction D2, the edge of the friction reducing layer 6 may extend beyond the edge of the absorbing part 4 in a direction away from the absorbing part 4. With this configuration, even if the back sheet 2 and the absorbing part 4 are misaligned with each other in the in-plane direction and the misalignment is not corrected immediately, the above-described effect of enabling the back sheet 2 and the absorbing part 4 to slide relative to each other in the longitudinal direction D1 and/or the width direction D2 can be maintained.

The static friction coefficient of the friction reducing layer 6 with the back sheet 2 is preferably less than or equal to 98%, more preferably less than or equal to 90%, still more preferably less than or equal to 75%, and further preferably less than or equal to 60% of the static friction coefficient of the absorbing part 4 with the back sheet 2 measured under the same conditions. Also, the static friction coefficient of the absorbing part 4 with the friction reducing layer 6 is preferably less than or equal to 90%, more preferably less than or equal to 80%, still more preferably less than or equal to 65%, and further preferably less than or equal to 50% of the static friction coefficient of the absorbing part 4 with the back sheet 2 measured under the same conditions.

The dynamic friction coefficient of the friction reducing layer 6 with the back sheet 2 is preferably less than or equal to 98%, more preferably less than or equal to 90%, still more preferably less than or equal to 75%, and further preferably less than or equal to 60% of the dynamic friction coefficient of the absorbing part 4 with the back sheet 2 measured under the same conditions. Also, the dynamic friction coefficient of the absorbing part 4 with the friction reducing layer 6 is preferably less than or equal to 80%, more preferably less than or equal to 50%, still more preferably less than or equal to 30%, further preferably less than or equal to 10%, and particularly preferably less than or equal to 5% of the dynamic friction coefficient of the absorbing part 4 with the back sheet 2 measured under the same conditions.

When the absorbing part 4 is enveloped in the enveloping sheet 45, the static friction coefficient and the dynamic friction coefficient of the absorbing part 4 with the friction reducing layer 6 may be represented by the static friction coefficient and the dynamic friction coefficient of the enveloping sheet 45 with the friction reducing layer 6.

When the absorbent article 1 is used, the friction between the friction reducing layer 6 and the back sheet 2 or the absorbing part 4 may be less than the friction between the skin and the absorbent article 1 (the friction between the skin and the top sheet 3).

In each of the examples illustrated in FIGs. 2 through 4, one friction reducing layer 6 is provided. However, two or more friction reducing layers 6 may be provided. In this case, two or more of the above-described configurations of the friction reducing layer 6 (FIGs. 2 through 4) may be combined in any appropriate manner.

The thickness of the friction reducing layer 6 may be about 50 to about 1,000 um. When the friction reducing layer 6 is a sheet-like part (FIGs. 2 and 4), the thickness of the sheet-like part (or a total thickness of multiple sheet-like parts) is preferably 100 to 1,000 um. Also, when the friction reducing layer 6 is implemented by the friction reduced portion 6a formed on at least one of the back sheet 2 and the absorbing part 4 (FIG. 3), the thickness is preferably 50 to 500 µm.

FIG. 5 illustrates a variation of the absorbent article according to the embodiment of the present invention. FIG. 5 illustrates a state in which the top sheet 2 and the side nonwoven fabrics 7 are removed from the absorbent article 1 of FIG. 1. FIG. 5 also illustrates antislip parts 9 disposed on the underwear side of the back sheet 3.

The antislip parts 9 may be adhesive or non-adhesive layers for fixing the absorbent article 1 to the underwear. The adhesive layers preferably include, for example, a styrene polymer, a tackifier, a plasticizer, or a combination of them as a main component. The antislip parts 9 implemented as non-adhesive layers may be formed of any material that increases the friction between the absorbent article 1 and an object to which the absorbent article 1 is attached. One of or a combination of materials including natural or synthetic rubbers as base polymers may be used for the non-adhesive layers.

In the example of FIG. 5, three strips of antislip parts 9 extending in the longitudinal direction D1 are provided. However, the shape and size of the antislip parts 9 and the region in which the antislip parts 9 are disposed are not limited to those illustrated in FIG. 5. The friction reducing layer 6 is preferably disposed to overlap the antislip parts 9 in plan view.

The portion where the antislip parts 9 are provided on the back sheet 2 is firmly fixed to the underwear, and therefore tends to deform along with the underwear when the wearer moves and to rub the crotch (or private parts) facing the absorbent article. However, with the configuration where the friction reducing layer 6 overlaps the antislip parts 9 in plan view, the back sheet 2 and the absorbing part 4 can smoothly slide at the overlapping positions in a direction along their contact surface. This configuration enables the absorbing part 4 and the top sheet 2 placed on the absorbing part 4 to smoothly follow the movement of the body and enables the back sheet 2 to follow the movement of the underwear.

The friction reducing layer 6 is preferably provided at least in the middle region M, the rear region R, or both of the middle region M and the rear region R. In the example illustrated in FIG. 5, the friction reducing layer 6 is provided across the middle region M and the rear region R, but not provided in the front region F. Here, the middle region M includes the body fluid outlet region A and corresponds to a particularly delicate or soft portion of the body. Therefore, it is possible to effectively improve the wear comfort by preventing friction in the middle region M.

Also, in the rear region R, the absorbent article tends to move due to the movement of the legs during, for example, walking. That is, each time the right or left leg is moved forward in the walking direction during walking, the underwear is pulled by the moved leg, and the absorbent article is pulled along with the underwear in the walking direction. Therefore, friction is likely to occur between the absorbent article and the skin facing the absorbent article. Accordingly, the wear comfort can be effectively improved by providing the friction reducing layer 6 at least in the rear region R to prevent friction. Also, because the rear region R is adjacent to the middle region M, the movement of the rear region R tends to be transmitted to the middle region M. Therefore, providing the friction reducing layer 6 in the rear region R makes it possible to prevent friction between the absorbent article and the skin not only in the rear region R but also in the middle region M in front of the rear region R.

The back sheet 2 may be partially bonded directly or via the friction reducing layer 6 to the absorbing part 4. The bonded portion between the back sheet 2 and the absorbing part 4 makes it possible to stabilize the shape of the absorbent article and makes it possible to prevent the entire absorbent article from being damaged even when the wearer makes hard movements.

When the back sheet 2 and the absorbing part 4 are partially bonded to each other, the plan-view area of the bonded portion may be less than or equal to 20%, preferably less than or equal to 10%, more preferably less than or equal to 5%, and still more preferably 1% of the plan-view area (100%) of the absorbing part 4. Further preferably, the back sheet 2 and the absorbing part 4 may not be directly or indirectly bonded to each other. Setting the plan-view area at a value less than or equal to 20% makes it possible to avoid preventing the sliding operation between the back sheet 2 and the absorbing part 4.

The back sheet 2 and the absorbing part 4 may be bonded to each other in at least a portion of the front region F. The front region F is less affected by the movement of the body, and the friction between the absorbent article 1 and the skin is less likely to occur in the front region F than in the middle region M and the rear region R. Therefore, bonding the back sheet 2 and the absorbing part 4 in at least a portion of the front region F makes it possible to improve the shape stability of the absorbent article 1 while enabling the relative movement between the back sheet 2 and the absorbing part 4 and thereby maintaining the effect of improving the wear comfort.

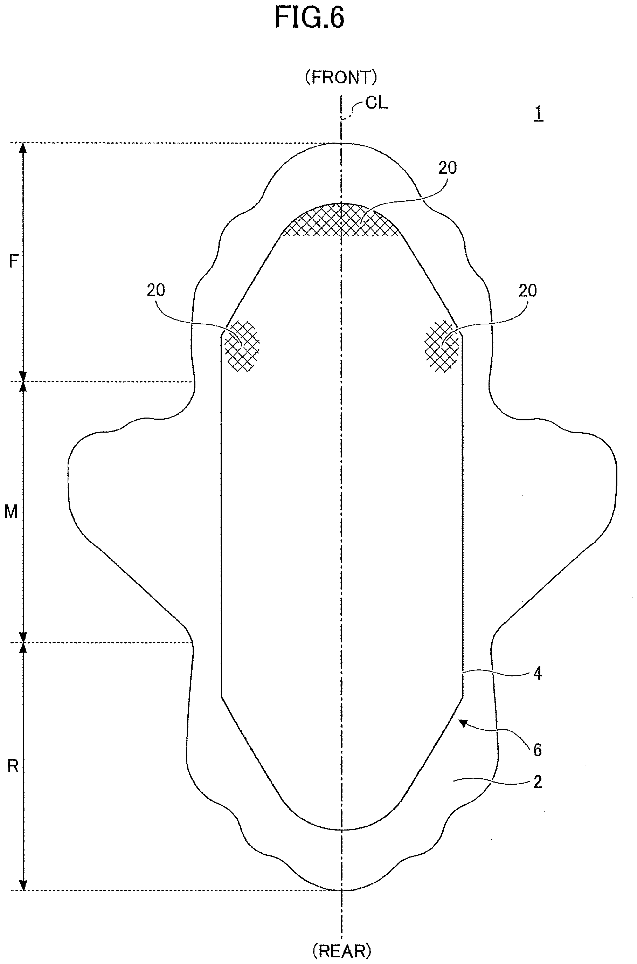

FIG. 6 illustrates examples of bonded portions between the back sheet 2 and the absorbing part 4. Similarly to FIG. 5, FIG. 6 illustrates a state in which the top sheet 2 and the side nonwoven fabrics 7 are removed from the absorbent article 1 of FIG. 1. In the example illustrated in FIG. 6, the absorbing part 4 and the friction reducing layer 6 are shaped and arranged such that their contours are aligned with each other in plan view. In FIG. 6, bonded portions 20 where the absorbing part 4 and the back sheet 2 are bonded via the friction reducing layer 6 are indicated by hatchings. As illustrated in FIG. 5, bonding the back sheet 2 and the absorbing part 4 to each other at the front end of the absorbing part 4 and/or front side-ends of the absorbing part 4 makes the absorbent article 1 less likely to be damaged and makes it possible to stabilize the shape of the absorbent article without reducing the function of the friction reducing layer 6 in the middle region M and the rear region R.

When the back sheet 2 and the absorbing part 4 are not bonded to each other as in the examples of FIGs. 1 through 3, the back sheet 2 and the absorbing part 4 can slide relative to each other across the entire absorbing part 4, and therefore the effect of preventing friction that may occur between the absorbent article 1 and the skin becomes high.

The present embodiment may be applied to a diaper in addition to a sanitary napkin, a pantiliner, an incontinence pad and so on. The present embodiment is particularly preferably applied to a sanitary napkin. Because a sanitary napkin is often used with its skin side placed in contact with the private parts, the movement of the wearer is more likely to cause friction between the absorbent article and the skin compared with other types of absorbent articles. Accordingly, using the present embodiment makes it possible to reduce the friction between an absorbent article and the skin and prevent the reduction in wear comfort. In some cases, the present embodiment can also prevent, for example, itchiness, pain, and rashes suffered by a wearer.

Preferred embodiments of the present invention are described below as appendices.

### (Appendix 1)

According to an embodiment of Appendix 1, an absorbent article includes a permeable top sheet, an impermeable back sheet, an absorbing part disposed between the top sheet and the back sheet, and a friction reducing layer disposed between the back sheet and the absorbing part. The friction between the friction reducing layer and the back sheet or the friction between the friction reducing layer and the absorbing part is less than the friction between the absorbing part and the back sheet.

According to the embodiment of Appendix 1, the friction reducing layer provided between the back sheet and the absorbing part can reduce the friction between the back sheet and the absorbing part. This configuration enables the back sheet and the absorbing part to slide relative to each other and thereby makes it possible to prevent the back sheet and the absorbing part and the top sheet placed on the absorbing part to move along with each other. Therefore, even when an underwear moves along with the movement of legs and the back sheet fixed to the underwear moves along with the movement of the underwear, the movement of the back sheet is less likely to be transmitted to the absorbing part and the top sheet, and the absorbing part and the top sheet can move along with the skin of the private parts contacting the absorbent article. This in turn makes it possible to suppress the movement of the absorbing part and the top sheet relative to the skin, and thereby makes it possible to prevent friction between the absorbent article and the skin.

### (Appendix 2)

According to an embodiment of Appendix 2, the absorbent article includes a middle region including a body fluid outlet region that faces a body fluid outlet of a wearer when the absorbent article is worn and a rear region disposed next to the rear end of the middle region, and the friction reducing layer is provided in at least one or both of the middle region and the rear region.

The embodiment of Appendix 2 makes it possible to prevent friction between the absorbent article and the skin in the middle region corresponding to the body fluid outlet and surrounding portions that are delicate and soft and/or makes it possible to specifically prevent friction between the absorbent article and the skin in the rear region where the absorbent article is particularly likely to move during, for example, walking.

### (Appendix 3)

According to an embodiment of Appendix 3, the friction reducing layer is a sheet-like part inserted between the back sheet and the absorbing part. A friction reducing treatment is applied to at least one surface of the sheet-like part.

The embodiment of Appendix 3 can reliably reduce the friction. Also, the configuration of the friction reducing layer can be changed according to the purpose of the absorbent article by just changing the sheet-like part to be inserted, i.e., without greatly changing the manufacturing process.

### (Appendix 4)

According to an embodiment of Appendix 4, the friction reducing layer is formed by applying a friction reducing treatment to at least one of the back sheet and the absorbing part.

The embodiment of Appendix 4 makes it possible to provide the friction reducing layer without increasing the number of components and thereby makes it possible to reduce the manufacturing costs. Also, this embodiment makes it possible to reduce the thickness of the entire absorbent article.

### (Appendix 5)

According to an embodiment of Appendix 5, the friction reducing treatment is a process of applying a material including one or more of a silicone resin, a fluororesin, and a polyacetal resin.

The embodiment of Appendix 5 makes it possible to improve the friction reducing effect of the friction reducing layer.

### (Appendix 6)

According to an embodiment of Appendix 6, the absorbent article further includes
an antislip part provided on the back sheet to fix the absorbent article to an underwear,
and the friction reducing layer is disposed to overlap the antislip part in plan view.

The embodiment of Appendix 6 makes it possible to stably fix the absorbent article to an underwear with the antislip part. Also, even in this case, the back sheet can be reliably slid relative to the absorbing part and the top sheet.

The present application is based on and claims priority to Japanese Patent Application No. 2018-182563 filed on September 27, 2018.

## Claims

1. An absorbent article (1), comprising:
a permeable top sheet (3);
an impermeable back sheet (2);
an absorbing part (4) disposed between the top sheet (3) and the back sheet (2); and
a friction reducing layer (6) disposed between the back sheet (2) and the absorbing part (4),
wherein the friction reducing layer (6) is at least one selected from a group consisting of: a sheet-like part inserted between the back sheet (2) and the absorbing part (4), a friction reducing treatment being applied to at least one surface of the sheet-like part; a sheet-like part that is inserted between the back sheet (2) and the absorbing part (4), and is formed of at least one material selected from a group consisting of silicone resin, fluororesin, or polyacetal resin; and a layer formed by applying the friction reducing treatment to at least one of the back sheet (2) and the absorbing part (4),
wherein the friction reducing treatment is a process of applying at least one of silicone resin, fluororesin, polycetal resin, talc, silica, and mica,
wherein friction between the friction reducing layer (6) and the back sheet (2) or friction between the friction reducing layer (6) and the absorbing part (4) is less than friction between the absorbing part (4) and the back sheet (2).

2. The absorbent article (1) as claimed in claim 1, wherein
the absorbent article (1) includes a middle region (M) including a body fluid outlet region (A) that faces a body fluid outlet of a wearer when the absorbent article (1) is worn and a rear region (R) disposed next to a rear end of the middle region (M); and
the friction reducing layer (6) is provided in at least one or both of the middle region (M) and the rear region (R).

3. The absorbent article (1) as claimed in claim 1, further comprising:
an antislip part (9) provided on the back sheet (2) to fix the absorbent article (1) to an underwear,
wherein the friction reducing layer (6) is disposed to overlap the antislip part (9) in plan view.

## Patentansprüche

1. Absorbierender Artikel (1), umfassend:
eine durchlässige obere Lage (3);
eine undurchlässige hintere Lage (2);
einen absorbierenden Teil (4), der zwischen der oberen Lage (3) und der hinteren Lage (2) angeordnet ist; und
eine reibungsmindernde Schicht (6), die zwischen der hintere Lage (2) und dem absorbierenden Teil (4) angeordnet ist,
wobei die reibungsmindernde Schicht (6) mindestens eine ist, die aus einer Gruppe ausgewählt ist, die besteht aus: einem folienähnlichen Teil, der zwischen der hintere Lage (2) und dem absorbierenden Teil (4) eingefügt ist, wobei eine reibungsmindernde Behandlung auf mindestens eine Oberfläche des folienähnlichen Teils aufgebracht wird; einem folienähnlichen Teil, der zwischen der hintere Lage (2) und dem absorbierenden Teil (4) eingefügt ist und aus mindestens einem Material gebildet ist, das aus einer Gruppe ausgewählt ist, die aus Silikonharz, Fluorharz oder Polyacetalharz besteht; und einer Schicht, die durch Aufbringen der reibungsmindernden Behandlung auf mindestens eine von der hintere Lage (2) und dem absorbierenden Teil (4) gebildet wird,
wobei die reibungsmindernde Behandlung ein Verfahren zum Auftragen von mindestens einem von Silikonharz, Fluorharz, Polyacetalharz, Talk, Siliciumdioxid und Glimmer ist,
wobei die Reibung zwischen der reibungsmindernden Schicht (6) und der hintere Lage (2) oder die Reibung zwischen der reibungsmindernden Schicht (6) und dem absorbierenden Teil (4) geringer ist als die Reibung zwischen dem absorbierenden Teil (4) und der hintere Lage (2).

2. Der absorbierende Artikel (1) nach Anspruch 1, wobei
der absorbierende Artikel (1) einen mittleren Bereich (M) mit einem Körperflüssigkeitsauslassbereich (A), der einem Körperflüssigkeitsauslass eines Trägers zugewandt ist, wenn der absorbierende Artikel (1) getragen wird, und einen hinteren Bereich (R) aufweist, der neben einem hinteren Ende des mittleren Bereichs (M) angeordnet ist; und
die reibungsmindernde Schicht (6) in mindestens einem oder beiden, dem mittleren Bereich (M) und dem hinteren Bereich (R), vorgesehen ist.

3. Absorbierender Artikel (1) nach Anspruch 1, ferner umfassend:
ein Antirutschteil (9), das auf der Rückenlage (2) vorgesehen ist, um den absorbierenden Artikel (1) an einer Unterwäsche zu befestigen,
wobei die reibungsmindernde Schicht (6) so angeordnet ist, dass sie den Antirutschteil (9) in der Draufsicht überlappt.

## Revendications

1. Article absorbant (1), comprenant :
une feuille supérieure (3) perméable ;
une feuille verso (2) imperméable ;
une partie absorbante (4) disposée entre la feuille supérieure (3) et la feuille verso (2) ; et
une couche de réduction de frottement (6) disposée entre la feuille verso (2) et la partie absorbante (4),
sachant que la couche de réduction de frottement (6) est au moins une couche sélectionnée dans un groupe constitué par : une partie de type feuille insérée entre la feuille verso (2) et la partie absorbante (4), un traitement de réduction de frottement étant appliqué à au moins une surface de la partie de type feuille ; une partie de type feuille qui est insérée entre la feuille verso (2) et la partie absorbante (4), et est composée d'au moins un matériau sélectionné dans un groupe constitué par une résine de silicone, une fluororésine, ou une résine de polyacétale ; et une couche formée en appliquant le traitement de réduction de frottement à au moins l'une de la feuille verso (2) et de la partie absorbante (4),
sachant que le traitement de réduction de frottement est un processus d'application d'au moins l'une d'une résine de silicone, d'une fluororésine, d'une résine de polyacétale, d'un talc, d'une silice, et d'un mica,
sachant que le frottement entre la couche de réduction de frottement (6) et la feuille verso (2) ou le frottement entre la couche de réduction de frottement (6) et la partie absorbante (4) est moindre que le frottement entre la partie absorbante (4) et la feuille verso (2).

2. L'article absorbant (1) tel que revendiqué dans la revendication 1, sachant que
l'article absorbant (1) inclut une zone médiane (M) incluant une zone de sortie de fluide corporel (A) qui fait face à une sortie de fluide corporel d'une porteuse lorsque l'article absorbant (1) est porté et une zone arrière (R) disposée près d'une extrémité arrière de la zone médiane (M) ; et
la couche de réduction de frottement (6) est prévue dans au moins l'une ou les deux de la zone médiane (M) et de la zone arrière (R).

3. L'article absorbant (1) tel que revendiqué dans la revendication 1, comprenant en outre :
une partie antidérapante (9) prévue sur la feuille verso (2) pour fixer l'article absorbant (1) à un sous-vêtement,
sachant que la couche de réduction de frottement (6) est disposée pour chevaucher la partie antidérapante (9) en vue en plan.
